(19) Europäisches Patentamt
European Patent Office
Office européen des brevets

(11) EP 0 678 497 B1

(12) FASCICULE DE BREVET EUROPEEN

(45) Date de publication et mention de la délivrance du brevet:
**21.01.1998 Bulletin 1998/04**

(51) Int Cl.[6]: **C07C 49/453**, C11B 9/00, A61K 7/46

(21) Numéro de dépôt: **95103221.8**

(22) Date de dépôt: **07.03.1995**

# (54) Composition contenant une quantité prépondérante de (1RS,5RS)-5-méthyl-exo-tricyclo[6.2.1.0 2,7]undécan-4-one et son utilisation en parfumerie

Zusammensetzung enthaltend einen überwiegenden Anteil an (1RS,5RS)-5-Methyl-exo-tricyclo[6.2.1.0 2,7]undecan-4-on und ihre Verwendung in der Parfümerie

Composition containing a preponderant amount of (1RS,5RS)-5-methyl-exo-tricyclo[6.2.1.0 2,7]undecan-4-one and its use in parfumery

(84) Etats contractants désignés:
**CH DE FR GB LI NL**

(30) Priorité: **22.04.1994 CH 1255/94**

(43) Date de publication de la demande:
**25.10.1995 Bulletin 1995/43**

(73) Titulaire: **FIRMENICH SA**
**1211 Genève 8 (CH)**

(72) Inventeurs:
- **Fankhauser, Peter**
  **CH-1217 Meyrin (CH)**
- **Fantini, Piero**
  **CH-1205 Geneve (CH)**
- **Blanc, Pierre-Alain**
  **CH-1263 Crassier (CH)**

(74) Mandataire:
**Salvaterra-Garcia, Maria de Lurdes et al**
**Firmenich SA**
**Département des Brevets**
**Case Postale 239**
**1211 Genève 8 (CH)**

(56) Documents cités:
**FR-A- 2 236 828**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 678 497 B1

## Description

La présente invention a trait au domaine de la parfumerie. Elle concerne, plus particulièrement, une composition constituée par un mélange de (1RS,5RS)-5-méthyl-exo-tricyclo[6.2.1.0$^{2,7}$]undécan-4-one et (1RS,5SR)-5-méthyl-exo-tricyclo[6.2.1.0$^{2,7}$]undécan-4-one, la première étant présente dans le mélange en quantité prépondérante.

L'art antérieur décrit que la 5-méthyl-tricyclo[6.2.1.0$^{2,7}$]undécan-4-one peut être utilisée en parfumerie pour développer le caractère floral des compositions dans lesquelles elle est incorporée, et pour exercer également une action fixative et renforçatrice (voir, par exemple, le brevet US 3,968,070).

Le brevet cité décrit aussi un procédé pour la préparation de ce composé, selon le schéma suivant:

SCHEMA I

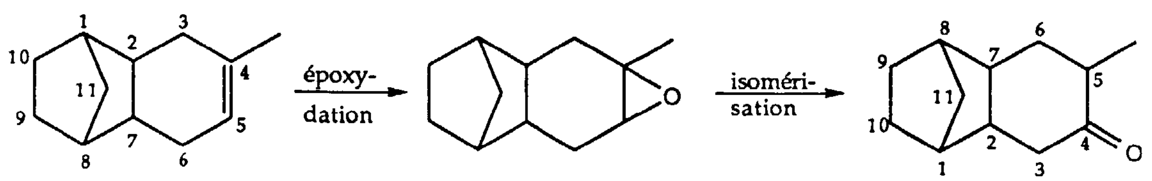


(III) (II) (I)

mais ne contient aucune indication sur la composition isomérique du produit obtenu. En reproduisant le procédé décrit, nous avons découvert que le produit obtenu était en fait un mélange contenant essentiellement la (1RS,5SR)-5-méthyl-exo-tricyclo[6.2.1.0$^{2,7}$]undécan-4-one, accompagnée d'une moindre quantité, de l'ordre de 20 à 25% en poids, de l'isomère (1RS,5RS) correspondant.

Du fait que le brevet US 3,968,070 est complètement muet en ce qui concerne la composition isomérique du produit obtenu, on est forcé d'admettre que ses auteurs n'ont pas reconnu le caractère distinctif de l'odeur de chacun des isomères individuels.

Or, nous avons maintenant découvert que la (1RS,5RS)-5-méthyl-exo-tricyclo[6.2.1.0$^{2,7}$]undécan-4-one, ayant la formule

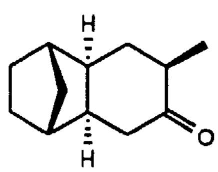

(Ia)

(1RS,5RS)

possède des propriétés odorantes tout à fait distinctes de celles de son isomère de configuration

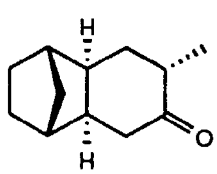

(Ib)

(1RS,5SR)

et distinctes de celles du produit décrit dans US 3,968,070.

Par ailleurs, grâce au procédé de l'invention décrit ci-après, il est maintenant possible d'obtenir des compositions riches en composé (Ia) dont les propriétés olfactives se révèlent être supérieures à celles des compositions de l'art antérieur, qui contenaient essentiellement le composé (Ib).

En effet, nous avons constaté que le composé (Ia) possède une odeur aromatique, à forte connotation thuyonique, très naturelle, légèrement camphrée et herbacée, qui évoque l'odeur du myrte et du cedarleaf. Il s'agit d'une note très puissante et tenace, aussi bien sur mouillette que sur du linge parfumé à l'aide de ce composé.

Pour sa part, le composé (Ib) possède une note nettement moins aromatique-thuyonique, et beaucoup plus camphrée, que celle du composé (Ia), avec une nette connotation fruitée, qui rappelle, par certains de ses aspects, la compote de rhubarbe. C'est une odeur moins puissante que celle du composé (Ia) et nettement moins élégante dans le caractère aromatique thuyonique particulièrement apprécié des parfumeurs.

Quoique les caractères olfactifs susmentionnés soient représentés en mieux chez le composé (Ia) pur ou pratiquement pur, les mêmes connotations, aromatique, thuyonique, myrte et cedarleaf, recherchées se retrouvent dans l'odeur des mélanges des deux isomères (Ia) et (Ib) qui contiennent une quantité prépondérante, c'est-à-dire plus de 50% en poids, du premier. Ces mélanges font également l'objet de l'invention et on peut citer à titre préférentiel les compositions constituées par au moins environ 75% en poids de (1RS,5RS)-5-méthyl-exo-tricyclo[6.2.1.$0^{2,7}$]undécan-4-one et une quantité déterminée, mais inférieure à environ 25% en poids, de (1RS,5SR)-5-méthyl-exo-tricyclo[6.2.1.$0^{2,7}$]undécan-4-one. De telles compositions peuvent être directement obtenues du procédé de l'invention et sont donc économiquement avantageuses par rapport au composé (Ia) pur, lequel exige une préparation chromatographique ou une distillation fractionnée.

Par comparaison avec le produit de l'art antérieur décrit dans le brevet US 3,968,070, constitué essentiellement de (1RS,5SR)-5-méthyl-exo-tricyclo[6.2.1.$0^{2,7}$]undécan-4-one, les compositions de l'invention permettent d'obtenir des effets aromatiques-thuyoniques beaucoup plus naturels et marqués, le composé décrit ayant tendance à amoindrir la fraîcheur de ces notes aromatiques de par sa connotation camphrée. L'effet olfactif obtenu avec le mélange de l'art antérieur est donc totalement distinct de celui produit par le composé (Ia) de l'invention et par les mélanges riches en ce composé, un résultat surprenant au vu de l'art antérieur et qui ressort clairement des exemples d'application présentés plus loin.

Selon l'invention, le composé (Ia) et les mélanges riches en ce composé peuvent être ajoutés aux compositions et articles que l'on désire parfumer dans des concentrations variées, qui dépendent de l'effet odorant voulu, ainsi que de la nature des autres ingrédients présents dans une composition parfumante donnée. On peut citer à titre d'exemple des concentrations de l'ordre de 1 à 10% ou même 20% en poids de composé ou composition selon l'invention, par rapport au poids de la base ou concentré parfumant auxquels ils sont ajoutés. Des concentrations inférieures aux valeurs citées seront normalement utilisées dans le parfumage d'articles de consommation divers tels les savons, les gels de douche ou bain, les shampoings ou après-shampoings, les préparations cosmétiques, les désodorisants corporels ou d'air ambiant, les détergents et adoucissants textiles ou les produits d'entretien.

Dans ces applications, la (1RS,5RS)-5-méthyl-exo-tricyclo[6.2.1.$0^{2,7}$]undécan-4-one et les compositions selon l'invention peuvent être utilisées seules ou en mélange avec d'autres ingrédients parfumants, des solvants ou des adjuvants d'usage courant en parfumerie.

La présente invention concerne également un procédé nouveau pour la préparation d'une composition contenant une quantité prépondérante de (1RS,5RS)-5-méthyl-exo-tricyclo[6.2.1.$0^{2,7}$]undécan-4-one, lequel procédé est caractérisé en ce qu'il comprend le traitement d'un mélange de (1RS,5RS)-5-méthyl-exo-tricyclo[6.2.1.$0^{2,7}$]undécan-4-one et de (1RS,5SR)-5-méthyl-exo-tricyclo[6.2.1.$0^{2,7}$]undécan-4-one, dans lequel cette dernière est en quantité prépondérante, avec une base forte, organique ou inorganique.

A titre de base forte on utilisera de préférence un alkoxyde ou un hydroxyde de métal alcalin, par exemple le méthylate de sodium ou l'hydroxyde de potassium. D'autres bases fortes convenant à cet usage sont les amines tertiaires, notamment la tributylamine.

La réaction peut se dérouler dans un solvant organique inerte tel que le toluène ou autre hydrocarbure, ou encore l'éther de pétrole. Alternativement, elle peut se dérouler sans solvant.

La réaction peut s'effectuer à une température variant de façon étendue et dont la valeur peut être facilement optimisée en fonction des conditions de réaction pour garantir une vitesse de réaction adaptée à l'utilisation à l'échelle industrielle.

Selon un mode d'exécution particulier, le procédé comprend une étape facultative selon laquelle on soumet ensuite la composition obtenue à une chromatographie préparative ou à une distillation fractionnée pour obtenir la (1RS,5RS)-5-méthyl-exo-tricyclo[6.2.1.$0^{2,7}$]undécan-4-one essentiellement pure, c'est-à-dire avec une pureté supérieure à 90%.

Le mélange de cétones utilisé comme produit de départ dans le procédé de l'invention peut être obtenu selon le schéma I, comme il est décrit dans le brevet US 3,968,070.

Comme il est cité dans ce brevet, l'époxydation du 4-méthyl-tricyclo[6.2.1.$0^{2,7}$]undéc-4-ène peut s'effectuer selon les techniques usuelles, notamment au moyen d'un peracide organique tel l'acide performique, peracétique, perbenzoïque, monoperbenzoïque, perphtalique ou trifluoroperacétique par exemple, en présence d'un solvant organique

inerte tel le chloroforme, le chlorure de méthylène, le trichloroéthylène, le dichloroéthane ou le toluène par exemple.

Ladite époxydation peut être en outre effectuée en milieu tamponné. On peut utiliser à cet effet un sel alcalin d'un acide organique comme l'acide formique, acétique, propionique, butyrique, oxalique, citrique, tartrique ou carbonique par exemple.

La réaction d'époxydation peut s'effectuer à une température variant de façon étendue et dont la valeur peut être facilement optimisée en fonction des conditions de réaction.

Les peracides organiques, utilisés pour ladite époxydation, peuvent être préparés in *situ*, par traitement d'un acide organique au moyen d'eau oxygénée, en présence d'un acide minéral, selon les techniques usuelles [voir par exemple: H. O. House, Modern Synthetic Reactions, Benjamin, Inc., New York (1965), p. 105 et suivantes].

La configuration isomérique exo du produit de départ est maintenue lors de cette époxydation. Le produit de cette époxydation est par ailleurs un mélange de deux isomères, représentés ci-après

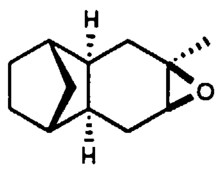

et

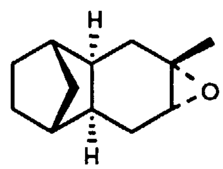

**(IIa)**
**~17%**
**(1RS,4SR)**

**(IIb)**
**~83%**
**(1RS,4RS)**

La composition du mélange susmentionné se révèle être pratiquement indépendante des conditions de réaction.

L'isomérisation de l'époxyde intermédiaire ainsi obtenu s'effectue par traitement dudit composé intermédiaire au moyen d'un agent isomérisant acide. Comme agent acide, on peut utiliser un acide minéral ou organique, ou encore un acide dit de Lewis tel que $BF_3$, $SnCl_4$, $FeCl_3$ ou $AlCl_3$ par exemple. On utilise de préférence le $BF_3$ en solution dans l'éther.

Alternativement, l'époxyde intermédiaire peut aussi être transformé dans la cétone désirée par traitement avec un catalyseur acide hétérogène tel une terre d'infusoires acide, l'alumine, le silice et autres.

Comme cité précédemment le produit de cette isomérisation est un mélange contenant une quantité prépondérante de (1RS,5SR)-5-méthyl-exotricyclo[6.2.1.$0^{2,7}$]undécan-4-one accompagnée de quantités inférieures de son isomère (1RS,5RS), et c'est ce mélange que l'on utilise comme produit de départ dans le procédé de la présente invention.

Le composé de départ (III) - voir Schéma I - peut être préparé par cycloaddition de Diels-Alder entre le norbornène et l'isoprène comme décrit dans le brevet US 3,968,070 à l'exemple 8a. La réaction y décrite fournit le 4-méthyl-tricyclo [6.2.1.$0^{2,7}$]undéc-4-ène exclusivement sous la forme exo.

L'invention sera maintenant décrite plus en détail à l'aide des exemples suivants, dans lesquels les températures sont indiquées en degrés centigrades et les abréviations ont le sens usuel dans l'art.

Exemple 1

Préparation de (1RS,5RS)-5-méthyl-exo-tricyclo[6.2.1.$0^{2,7}$]undécan-4-one

a) 4-Méthyl-exo-tricyclo[6.2.1.$0^{2,7}$]undéc-4-ène

Ce composé a été préparé de façon identique à celle décrite à l'exemple 8 du brevet US 3,968,070 par réaction du norbornène avec isoprène à une température d'environ 150°. Après le traitement usuel, on a obtenu le produit désiré pur à 99%, dont les caractères analytiques étaient les suivants :

| | |
|---|---|
| RMN($^1$H, 360MHz) : | 5,48(m, 1H); 2,20-1,95(m, 2H) ; 1,90(s large, 2H) ; 1,69(s, 3H); 1,65-1,41(m, 7H); 1,18(dd, J=7Hz, 2,5Hz, 2H); 1,0(d, J=11Hz, 1H) δppm |
| RMN($^{13}$C, 90MHz) : | 136,8(s); 121,5(d); 43,4(2d) ; 43,3(d); 43,2(d); 33,4(2t) ; 29,9(t); 29,8(t); 28,4(t); 23,3(q) δppm |
| SM : | 162(M$^+$, 57), 147(23), 134(38), 133(36), 119(25), 106(24), 105(32), 95(30), 94(63), 93(63), 92(41), 91(70), 79(100), 78(20), 77(49), 67(41), 66(82), 65(26), 53(19), 41(31), 39(27) |

b) (1RS,4RS)-4,5-Epoxy-4-méthyl-exo-tricylo[6.2.1.0$^{2,7}$]undécane

On a fait réagir le composé préparé sous a) avec de l'acide peracétique à 40%, dans le chlorure de méthylène, de façon identique à celle décrite à l'exemple 8 du brevet US 3,968,070. Après le traitement y décrit, on a obtenu un produit contenant environ 83% en poids du composé sous titre, accompagné d'environ 17% en poids de (1RS, 4SR)-4,5-époxy-4-méthyl-exo-tricydo[6.2.1.0$^{2,7}$]undécane.
Les caractères analytiques des deux isomères étaient les suivants:

A. (1RS,4SR)-4,5-époxy-4-méthyl-exo-tricyclo[6.2.1.0$^{2,7}$]undécane (17%):

| | |
|---|---|
| RMN($^{13}$C, 90MHz) signaux visibles: | 57,4(d); 56,2(s); 41,9(d); 41,7(d); 40,7(d); 40,6(d); 34,7(t); 32,0 (t); 29,6(2t); 21,9(q) δppm |
| SM : | 178(M$^+$, 24), 163(12), 150(40), 149(30), 136(39), 134(17), 121 (15), 119(15), 111(23), 109(22), 108(22), 107(30), 106(22), 95 (30), 94(23), 93(52), 92(43), 91(42), 82(25), 81(66), 80(38), 79 (76), 77(37), 67(100), 66(47), 55(30), 53(22), 43(52), 41(48), 39 (28) |

B. (1RS,4RS)-4,5-époxy-4-méthyl-exo-tricydo[6.2.1.0$^{2,7}$]undécane (83%):

| | |
|---|---|
| RMN($^1$H, 360MHz) : | 2,90(d, J=3,6Hz, 1H); 2,15(m, 1H); 1,95(dd, J=14Hz, 7Hz, 1H); 1,82(s large, 2H); 1,71-1,32(m, 7H); 1,26(s, 3H); 1,21-1,12(m, 2H); 1,05-0,96(m, 1H) δppm |
| RMN($^{13}$C, 90MHz) : | 57,8(d) ; 55,6(s) ; 42,1(d); 42,0(d) ; 39,8(d) ; 38,0(d); 33,2(t); 32,8(t); 29,4(t); 29,3(t); 28,0(t); 22,5(q) δppm |
| SM : | 178(M$^+$, 23), 163(15), 150(32), 149(77), 136(29), 134(15), 121(17), 119(18), 111(26), 109(21), 107(30), 95(26), 94(20), 93(56), 92(48), 91(50), 82(19), 81(81), 80(34), 79 (69), 77(36), 67(100), 66(38), 65(21), 55(28), 43(51), 41(58), 39(27) |

Ce mélange d'époxydes possède des propriétés odorantes utiles, développant une note ambrée, qui rappelle celle développée par l'ambrinol, aromatique et fruitée, de type melon.

c) (1RS,5SR)-5-Méthyl-exo-tricyclo[6.2.1.0$^{2,7}$]undécan-4-one

On a isomérisé le mélange d'époxydes décrit sous b) à l'aide d'éthérate de trifluorure de bore, suivi de bicarbonate de sodium, comme il est décrit dans le brevet américain cité plus haut.
Après le traitement usuel, le produit obtenu était un mélange contenant environ 80% en poids de (1RS,5SR)-5-méthyl-exo-tricyclo[6.2.1.0$^{2,7}$]undécan-4-one et 20% en poids de son isomère (1RS,5RS).
Les caractères analytiques de ce mélange coïncidaient avec ceux indiqués dans le brevet US 3,968,070.

d) Dans un ballon à 4 cols de 3l, équipé d'un agitateur magnétique, d'une colonne Vigreux de 15 cm et d'un thermomètre, on a chargé 1400 g du mélange obtenu selon c) dans 1000 g de toluène. On a ajouté tout à la fois 13,0 g (0,072 mole) de méthylate de sodium à 30% dans le méthanol. Le mélange réactionnel est devenu orange et la réaction était légèrement exothermique. On a maintenu sous agitation à température ambiante pendant 50 minutes.

On a ajouté ensuite de l'acide acétique glacial (4,3 g; 0,072 mole) et le mélange est redevenu jaune. On a distillé le solvant d'abord sous pression atmosphérique, puis sous vide partiel pour obtenir un produit brut (1405 g) dont la purification par distillation a fourni un mélange à 96% pur contenant environ 78% de (1RS,5RS)-5-méthyl-exo-tricyclo[6.2.1.0$^{2,7}$]undécan-4-one et 22% de son isomère (1RS,5SR).

Ce mélange possédait les propriétés olfactives désirées décrites dans l'introduction, c'est-à-dire une odeur de type aromatique, thuyonique, herbacé, rappelant le myrte et le cedarleaf.

Les deux stéréoisomères contenus dans le mélange ont été séparés soit par chromatographie préparative (colonne de silice: 1kg ; solvant cyclohexane/tert-butyl méthyl éther 95/5), soit par distillation fractionnée sur colonne à multiples plateaux de type Fischer Spaltrohr® de 1 m, sous vide. On a ainsi obtenu les composés suivants:

A. (1RS,5RS)-5-méthyl-exo-tricyclo[6.2.1.0$^{2,7}$]undécan-4-one :

Pureté : 94%
P. Eb. 96°/3x10$^2$ Pa

RMN($^1$H, 360MHz): 2,32-1,92(m, 5H); 1,87-1,70(m, 3H); 1,67-1,49(m, 3H); 1,40-1,10(m, 4H); 1,08(d,

| | |
|---|---|
| | J=7Hz, 3H) δppm |
| RMN($^{13}C$, 90MHz) : | 216,1(s); 43,0(d); 42,6(2d) ; 41,8(d); 41,5(t); 39,9(d); 33,8(t); 32,4(t); 29,6(t); 28,8(t); 16,0(q) δppm |
| SM : | 178($M^+$, 37), 150(52), 136(83), 135(17), 134(29), 121(18), 119(14), 109(33), 108(30), 107(36), 106(35), 95(42), 94(31), 93(54), 91(36), 82(48), 81(73), 80(40), 79(79), 77 (34), 67(100), 66(41), 55(30), 53(24), 41(38) |

B. (1RS,5SR)-5-méthyl-exo-tricyclo[6.2.1.0$^{2,7}$]undécan-4-one :

Pureté : 95%

| | |
|---|---|
| RMN($^{1}H$, 360MHz) : | 2,42-2,13(m, 3H); 1,98(s large, 2H); 1,88-1,50(m, 7H); 1,40-1,02(m, 3H); 1,12(d, J=7Hz, 3H)δppm |
| RMN($^{13}C$, 90MHz) : | 216,7(s) ; 42,7(d) ; 42,3(t); 41,7(d); 41,0(d) ; 40,3(d); 37,6(d); 32,4(t); 31,8(t); 29,7(t); 28,9(t); 16,1(q)δppm |
| SM : | 178($M^+$, 36), 150(55), 136(62), 134(20), 121(18), 109(26), 108(26), 107(28), 106(27), 95(26), 94(20), 93(38), 91(27), 82(32), 81(56), 80(34), 79(75), 73(34), 68(22), 67(100), 66(41), 65(21), 55(32), 53(25), 41(56), 39(31) |

Exemple 2

Composition parfumante

On a préparé une composition parfumante de base avec les ingrédients suivants :

| Ingrédients | Parties en poids |
|---|---|
| Exolide® 1) | 5 |
| Géraniate de méthyle | 3 |
| Essence de géranium synth. | 35 |
| Essence de lavandin | 30 |
| Mousse de chêne absolue à 10% * | 15 |
| Salicylate d'amyle | 7 |
| Total | 95 |

* dans le dipropylèneglycol (DIPG)

1) pentadécanolide; origine : Firmenich SA, Genève, Suisse

Avec cette composition de base de type fougère on a préparé une composition A par addition de 5 parties en poids du produit de l'art antérieur riche en (1RS,5SR)-5-méthyl-exo-tricyclo[6.2.1.0$^{2,7}$]undécan-4-one, décrit à l'exemple 1c), et une nouvelle composition B obtenue par addition de 5 parties en poids du mélange selon l'invention, riche en (1RS, 5RS)-5-méthyl-exo-tricyclo[6.2.1.0$^{2,7}$]undécan4-one, décrit dans l'exemple 1d).

Les compositions A et B ont ensuite été évaluées à l'aveugle par un panel d'experts parfumeurs, lesquels ont préféré à l'unanimité la composition B pour son odeur plus naturelle dans la note aromatique, ainsi que pour sa connotation herbacée plus puissante.

Par ailleurs, une composition nouvelle préparée par adjonction à la composition de base de 5 parties en poids de (1RS,5RS)-5-méthyl-exo-tricyclo[6.2.1.0$^{2,7}$]undécan-4-one pure à 94% a également été préférée par les parfumeurs. A leur avis, l'adjonction de ce composé renforçait encore plus l'effet olfactif aromatique, thuyonique, herbacé déjà observé avec le mélange riche en ce composé.

Exemple 3

Composition parfumante pour shampoing

On a préparé une composition parfumante de base, destinée au parfumage d'un shampoing, en mélangeant les ingrédients suivants:

| Ingrédients | Parties en poids |
|---|---|
| Acétate de styrallyle | 25 |
| Dodécanal à 50% * | 10 |
| Aldéhyde hexylcinnamique | 20 |
| Méthyl-nonyl-acétaldéhyde à 10% * | 30 |
| Allyl amyl glycolate à 10% * | 20 |
| 4-Nonalide 1) à 10% * | 5 |
| 1-Carvone | 70 |
| Citronellol | 140 |
| Cyclohexylpropionate d'allyle | 5 |
| Dihydromyrcenol 2) | 110 |
| Hedione ® 3) | 60 |
| Geranyl nitrile | 5 |
| Lilial ® 4) | 15 |
| Linalol | 120 |
| Menthol | 30 |
| Nerol | 35 |
| Essence de Petitgrain | 60 |
| Alcool phényléthylique | 100 |
| Essence d'orange douce | 40 |
| Salicylate d'hexyle | 10 |
| Salicylate de (Z)-3-hexényle | 5 |
| Terpinéol | 15 |
| Tonalide ® 5) | 25 |
| Vert de lilas 6) | 15 |
| Zestover ® 7) | 10 |
| Total | 980 |

* dans le dipropylèneglycol (DIPG)

1) origine: Firmenich SA, Genève, Suisse

2) 2,6-diméthyl-7-octén-2-ol ; origine: International Flavors & Fragrances, USA

3) dihydrojasmonate de méthyle ; origine: Firmenich SA, Genève, Suisse

4) 3-(4-tert-butyl-1-phényl)-2-méthylpropanal ; origine : Givaudan-Roure, Vernier, Suisse

5) 7-acétyl-1,1,3,4,4,6-tétraméthyltétraline; origine: PFW, Hollande

6) (2,2-diméthoxyéthyl)-benzène; origine: Givaudan-Roure, Vernier, Suisse

7) 2,4-diméthyl-3-cyclohexène-1-carbaldéhyde ; origine: Firmenich SA, Genève, Suisse

A cette composition de base de type aromatique, fleuri, vert, hespéridé on a ajouté 20 parties en poids du mélange de cétones selon l'invention préparé comme il est décrit à l'exemple 1d) pour obtenir une nouvelle composition A, et la même quantité du mélange connu contenant essentiellement la (1RS,5SR)-5-méthyl-exo-tricyclo[6.2.1.0$^{2,7}$]undécan-4-one pour obtenir une composition B.

Lorsqu'un panel constitué par dix experts parfumeurs a comparé à l'aveugle les compositions A et B, il est apparu que l'odeur de la composition A avait beaucoup plus de montant et de volume, et un caractère plus naturel que celle de la composition B. De l'avis des parfumeurs, l'addition du mélange de l'invention avait pour effet d'exalter en particulier le caractère conféré par la l-carvone, alors que l'addition du mélange de l'art antérieur, de par sa note camphrée, en estompait la fraîcheur.

Exemple 4

Parfumage de textiles

On a ajouté à une base non parfumée d'adoucissant textile du commerce 0,1% en poids de (1RS,5RS)-5-méthyl-exo-tricyclo[6.2.1.0$^{2,7}$]undécan-4-one, ou d'un mélange selon l'invention contenant environ 80% en poids de ce composé, pour obtenir un adoucissant parfumé, à l'aide duquel on a ensuite traité un lot standard de textiles lors d'un cycle

de lavage dans une machine à laver le linge.

Les textiles ont ensuite été évalués par un panel de 4 experts parfumeurs, à la sortie de la machine et après le séchage. A leurs avis, les textiles développaient une forte et agréable odeur de type aromatique, thuyonique et herbacé rappelant l'odeur du myrte et du cedarleaf.

Ce lot de textiles a été comparé à l'aveugle avec un autre lot standard de textiles de façon similaire mais en présence d'un adoucissant textile parfumé à l'aide du mélange connu contenant essentiellement l'isomère (1RS,5SR) de la cétone susmentionnée.

De l'avis des parfumeurs, ce dernier lot de textiles développait une odeur beaucoup moins fraîche et plus camphrée que celle du premier lot, auquel allait leur préférence. Par ailleurs, ils ont ensuite constaté que l'odeur du premier lot, quoique distincte de celle du second lot, était aussi beaucoup plus puissante et restait sur les tissus plus longtemps que celle du second lot.

**Revendications**

1. Composition constituée par un mélange de (1RS,5RS)-5-méthyl-exo-tricyclo[6.2.1.0$^{2,7}$]undécan-4-one et (1RS, 5SR)-5-méthyl-exo-tricyclo[6.2.1.0$^{2,7}$]undécan-4-one, la première étant présente dans le mélange en quantité prépondérante.

2. Composition selon la revendication 1 constituée par une quantité égale ou supérieure à 75% en poids de (1RS, 5RS)-5-méthyl-exo-tricyclo[6.2.1.0$^{2,7}$]undécan-4-one et une quantité déterminée, mais égale ou inférieure à 25% en poids, de (1RS,5SR)-5-méthyl-exo-tricyclo[6.2.1.0$^{2,7}$]undécan-4-one.

3. Composition selon la revendication 2, constituée par environ 78% de (1RS,5RS)-5-méthyl-exo-tricyclo[6.2.1.0$^{2,7}$] undécan-4-one et environ 22% de (1RS,5SR)-5-méthyl-exo-tricyclo[6.2.1.0$^{2,7}$]undécan-4-one.

4. (1RS,5RS)-5-Méthyl-exo-tricyclo[6.2.1.0$^{2,7}$]undécan-4-one ayant une pureté supérieure à 90%.

5. Utilisation d'une composition selon l'une des revendications 1 à 3 à titre d'ingrédient parfumant pour la préparation de compositions parfumantes ou articles parfumés.

6. Utilisation de la (1RS,5RS)-5-méthyl-exo-tricyclo[6.2.1.0$^{2,7}$]undécan-4-one selon la revendication 4, à titre d'ingrédient parfumant pour la préparation de compositions parfumantes ou articles parfumés.

7. Composition parfumante ou article parfumé résultant de l'utilisation selon la revendication 5 ou 6.

8. Article parfumé selon la revendication 7, sous forme d'un parfum ou d'une eau de toilette, d'un savon, d'un gel de douche ou bain, d'un shampoing ou d'un après-shampoing, d'une préparation cosmétique, d'un désodorisant corporel ou d'air ambiant, d'un détergent ou adoucissant textile, ou d'un produit d'entretien.

9. Procédé pour la préparation d'une composition selon la revendication 1, caractérisé en ce qu'il comprend le traitement d'un mélange de (1RS,5RS)-5-méthyl-exo-tricyclo[6.2.1.0$^{2,7}$]undécan-4-one et de (1RS,5SR)-5-méthyl-exo-tricyclo[6.2.1.0$^{2,7}$]undécan-4-one, dans lequel cette dernière est en quantité prépondérante, avec une base forte, organique ou inorganique.

10. Procédé selon la revendication 9, caractérisé en ce que la base forte est un alkoxyde ou un hydroxyde de métal alcalin.

11. Procédé selon la revendication 9, caractérisé en ce qu'on traite un mélange contenant environ 80% en poids de (1RS,5SR)-5-méthyl-exo-tricyclo[6.2.1.0$^{2,7}$]undécan-4-one et environ 20% en poids de (1RS,5RS)-5-méthyl-exo-tricyclo[6.2.1.0$^{2,7}$]undécan-4-one, avec du méthylate de sodium pour obtenir une composition contenant environ 78% en poids de (1RS,5RS)-5-méthyl-exo-tricydo[6.2.1.0$^{2,7}$]undécan-4-one et 22% en poids de (1RS,5SR)-5-méthyl-exo-tricydo[6.2.1.0$^{2,7}$]undécan-4-one.

12. Procédé selon l'une des revendications 9 à 11, caractérisé en ce qu'on soumet ensuite la composition obtenue à une chromatographie préparative ou à une distillation fractionnée pour obtenir la (1RS,5RS)-5-méthyl-exo-tricyclo [6.2.1.0$^{2,7}$]undécan-4-one essentiellement pure.

## Patentansprüche

1. Zusammensetzung, welche aus einer Mischung von (1RS,5RS)-5-Methyl-exo-tricyclo[6.2.1.$0^{2,7}$]undecan-4-on und (1RS,5SR)-5-Methyl-exo-tricyclo[6.2.1.$0^{2,7}$]undecan-4-on besteht, wobei das erstere in der Mischung mengenmäßig überwiegend vorliegt.

2. Zusammensetzung gemäß Anspruch 1, welche aus einer Menge von gleich oder mehr als 75 Gew.-% (1 RS,5RS)-5-Methyl-exo-tricyclo[6.2.1.$0^{2,7}$]undecan-4-on und einer bestimmten Menge von (1RS,5SR)-5-Methyl-exo-tricydo [6.2.1.$0^{2,7}$]undecan-4-on, welche jedoch gleich oder weniger als 25 Gew.-% beträgt, besteht.

3. Zusammensetzung gemäß Anspruch 2, welche aus ca. 78% (1RS,5RS)-5-Methyl-exo-tricyclo[6.2.1.$0^{2,7}$]undecan-4-on und ca. 22% (1RS,5SR)-5-Methyl-exo-tricyclo[6.2.1.$0^{2,7}$]undecan-4-on besteht.

4. (1RS,5RS)-5-Methyl-exo-tricyclo[6.2.1.$0^{2,7}$]undecan-4-on mit einer Reinheit von mehr als 90%.

5. Verwendung einer Zusammensetzung gemäß einem der Ansprüche 1 bis 3 als Riechstoffbestandteil bei der Herstellung von Duftzusammensetzungen bzw. parfümierten Artikeln.

6. Verwendung des (1RS,5RS)-5-Methyl-exo-tricyclo[6.2.1.$0^{2,7}$]undecan-4-on gemäß Anspruch 4 als Riechstoffbestandteil bei der Herstellung von Duftzusammensetzungen bzw. parfümierten Artikeln.

7. Duftzusammensetzung bzw. parfümierter Artikel, der aus der Verwendung gemäß Anspruch 5 oder 6 resultiert.

8. Parfümierter Artikel gemäß Anspruch 7 in Form von Parfüm oder Eau de Toilette, Seife, Dusch- bzw. Badegel, Shampoo oder eines nach der Haarwäsche zu verwendenden Pflegemittels, eines kosmetischen Präparates, von Körper- oder Raumluftdeodorant, eines Reinigungsmittels oder Weichspülers für Textilien oder eines Universalhaushaltsreiniger.

9. Verfahren zur Herstellung einer Zusammensetzung gemäß Anspruch 1, dadurch gekennzeichnet, daß es die Behandlung einer Mischung aus (1RS,5RS)-5-Methyl-exo-tricyclo[6.2.1.$0^{2,7}$]undecan-4-on und (1RS,5SR)-5-Methyl-exo-tricyclo[6.2.1.$0^{2,7}$]undecan-4-on umfaßt, bei der das letztere mengenmäßig überwiegt, mit einer starken organischen oder anorganischen Base.

10. Verfahren gemäß Anspruch 9, dadurch gekennzeichnet, daß die starke Base ein Alkoxid oder ein Hydroxid eines Alkalimetalls ist.

11. Verfahren gemäß Anspruch 9, dadurch gekennzeichnet, daß eine Mischung, welche ca. 80 Gew.-% (1RS,5SR)-5-Methyl-exo-tricyclo[6.2.1.$0^{2,7}$]undecan-4-on und ca. 20 Gew.-% (1RS,5RS)-5-Methyl-exo-tricyclo[6.2.1.$0^{2,7}$]undecan-4-on enthält, mit Natriummethylat behandelt wird, um eine Zusammensetzung herzustellen, welche ca. 78 Gew.-% (1RS,5RS)-5-Methyl-exo-tricyclo[6.2.1.$0^{2,7}$]undecan-4-on und 22 Gew.-% (1RS,5SR)-5-Methyl-exo-tricyclo[6.2.1.$0^{2,7}$]undecan-4-on enthält.

12. Verfahren gemäß einem der Ansprüche 9 bis 11, dadurch gekennzeichnet, daß die hergestellte Zusammensetzung daraufhin einer präparativen Chromatographie oder einer fraktionierten Destillation unterzogen wird, um im wesentlichen reines (1RS,5RS)-5-Methyl-exo-tricyclo[6.2. 1.$0^{2,7}$]undecan-4-on herzustellen.

## Claims

1. A composition formed of a mixture of (1RS,5RS)-5-methyl-exo-tricyclo[6.2.1.$0^{2,7}$]undecan-4-one and (1RS,5SR)-5-methyl-exo-tricyclo[6.2.1.$0^{2,7}$] undecan-4-one, wherein the former is present in the mixture in a preponderant amount.

2. A composition according to claim 1, consisting of 75% by weight or more of (1RS,5RS)-5-methyl-exo-tricyclo [6.2.1.$0^{2,7}$]undecan-4-one and a certain amount of (1RS,5SR)-5-methyl-exo-tricyclo[6.2.1.$0^{2,7}$] undecan-4-one, the latter amount being equal or less than 25% by weight.

3. The composition of claim 2, which consists of about 78% by weight of (1RS,5RS)-5-methyl-exo-tricyclo[6.2.1.$0^{2,7}$]

undecan-4-one and about 22% by weight of (1RS,5SR)-5-methyl-exo-tricyclo[6.2.1.$0^{2,7}$]undecan-4-one.

4. (1RS,5RS)-5-Methyl-exo-tricyclo[6.2.1.$0^{2,7}$]undecan-4-one having a purity above 90%.

5. Use of a composition according to any one of claims 1 to 3 as a perfuming ingredient for the preparation of perfuming compositions or perfumed articles.

6. Use of (1RS,5RS)-5-methyl-exo-tricyclo[6.2.1.02,7]undecan-4-one according to claim 4, as a perfuming ingredient for the preparation of perfuming compositions or perfumed articles.

7. A perfuming composition or a perfumed article resulting from the use according to claim 5 or 6.

8. A perfumed article according to claim 7, in the form of a perfume or a cologne, a soap, a bath or shower gel, a shampoo or a hair conditioner, a cosmetic preparation, a body deodorant, an air freshener, a detergent or a fabric softener, or a household product.

9. A process for the preparation of a composition according to claim 1, which comprises treating a mixture of (1RS, 5RS)-5-methyl-exo-tricyclo[6.2.1.$0^{2,7}$]undecan-4-one and (1RS,5SR)-5-methyl-exo-tricyclo[6.2.1.$0^{2,7}$] undecan-4-one, wherein the latter is present in a preponderant amount, with a strong organic or inorganic base.

10. The process according to claim 9, wherein the strong base is an alkali metal alkoxide or hydroxide.

11. The process according to claim 9, wherein a mixture containing about 80% by weight of (1RS,5SR)-5-methyl-exo-tricyclo[6.2.1.$0^{2,7}$]undecan-4-one and about 20% by weight of (1RS,5RS)-5-methyl-exo-tricyclo[6.2.1.$0^{2,7}$]undecan-4-one is treated with sodium methylate, to thereby provide a composition containing about 78% by weight (1RS,5RS)-5-methyl-exo-tricyclo[6.2.1.$0^{2,7}$]undecan-4-one and about 22% by weight of (1RS,5SR)-5-methyl-exo-tricyclo[6.2.1.$0^{2,7}$]undecan-4-one.

12. The process according to any one of claims 9 to 11, wherein the composition is further subjected to preparative chromatography or to fractional distillation to obtain essentially pure (1RS,5RS)-5-methyl-exo-tricyclo[6.2.1.$0^{2,7}$] undecan-4-one.